# DEMANDE DE BREVET EUROPEEN

(11) **EP 0 764 724 A1**
(43) Date de publication de la demande: **26.03.1997**
(21) Numéro de dépôt: 96401798.2
(22) Date de dépôt: 20.08.1996
(51) Int. Cl.: C12N 15/74, C12N 1/20, C12N 1/21

(54) **Séquences d'acides nucléiques et plasmides comprenant au moins un mécanisme de résistance aux phages, bactéries les contenant et leur utilisation**

(30) Priorité: 22.08.1995 FR 9509980
(71) Demandeur: SYSTEMS BIO-INDUSTRIES, F-92100 Boulogne (FR)
(72) Inventeur: Prevots, Fabien, 31400 Toulouse (FR); Tolou, Sandrine, 31650 St Orens de Gameville (FR); Daloyau, Marlène, 31320 Castanet (FR)
(74) Mandataire: Gillard, Marie-Louise

(57) **Abrégé**

L'invention a pour objet une séquence d'ADN d'environ 1345 pb comprenant au moins un mécanisme de résistance aux phages obtenue à partir de la séquence d'ADN 3704 pb contenue dans la souche de Lactococcus lactis ssp diacetylactis déposée à la C.N.C.M. sous le N° I-941 le 12 Avril 1990. L'invention a également pour objet la séquence d'ADN de 3704 pb.

Application: bactéries, notamment lactiques résistantes aux phages.

LISTE DE SEQUENCES

(1) INFORMATION GENERALE:

(i) DEPOSANT:
(A) NOM: SYSTEMS BIO-INDUSTRIES
(B) RUE: 4, Place des Ailes
(C) VILLE: Boulogne
(E) PAYS: France
(F) CODE POSTAL: 92100
(G) TELEPHONE: 47 12 25 25
(H) TELECOPIE: 47 12 26 56

(ii) TITRE DE L' INVENTION: Séquences d'acides nucléiques et
plasmides comprenant au moins un mécanisme de résistance
aux phages, bactéries les contenant et leur utilisation

(iii) NOMBRE DE SEQUENCES: 8

(iv) FORME DECHIFFRABLE PAR ORDINATEUR:
(A) TYPE DE SUPPORT: Floppy disk
(B) ORDINATEUR: IBM PC compatible
(C) SYSTEME D' EXPLOITATION: PC-DOS/MS-DOS
(D) LOGICIEL: PatentIn Release #1.0, Version #1.30 (OEB)

(vi) DONNEES DE LA DEMANDE ANTERIEURE:
(A) NUMERO DE LA DEMANDE: FR 95 09980
(B) DATE DE DEPOT: 22-AUG-1995

(2) INFORMATION POUR LA SEQ ID NO: 1:

(i) CARACTERISTIQUES DE LA SEQUENCE:
(A) LONGUEUR: 3704 paires de bases
(B) TYPE: acide nucléique
(C) NOMBRE DE BRINS: simple
(D) CONFIGURATION: linéaire

(ii) TYPE DE MOLECULE: ADN (génomique)

(iii) HYPOTHETIQUE: NON

(iii) ANTI-SENS: NON

(vi) ORIGINE
(A) ORGANISME : Lactococcus lactis

(ix) CARACTERISTIQUE ADDITIONELLE:
(A) NOM/CLE: CDS
(B) EMPLACEMENT: 1095..2132

(xi) DESCRIPTION DE LA SEQUENCE: SEQ ID NO: 1:

GATCAAAACT GATGCAAATG GAGTAATTAA GGCAAATCAT CTAAAACCAG GTAAATACAC 60
TTTTGTTGAA ACAGAGGCTC CAGCAGGTTA TCAATTAAGT CAAGAAACTA GAGCTTTTGA 120
AATTAAAGCA AGCGCAGAAA ACAAACCACA AGTTGTTAAT ACTGGAAAGT TCGTCAATAA 180
AAGACTTCCA ATTACACCTA AAAAGCCAGA ATTACCTAAG ACAGGAGAAG AACGAAATAC 240
TTTCTTGCCT ATAGTTGGTG TTGGACTATT AATGGTGGGT GCTACTTTAT ATGTATTTTT 300
TAAAAGACGT AAAATGTAAA ATTATATAAA AACATGGCTT TAGCCATGTT TTTGATTTGG 360
GAAAAGTGGT AGTGACTTAA TACAAAAATA TTTTAGTTTG TAATACCTAA AAATAATACT 420
TCAGTTTGCA GTATTCCAAT AAGTAAAAAA GATTTGGCTA TATTAGAACC ACCCAAAAAA 480
ACTCAAAATC ATGTAAATAT TAAGCTTGGA TTGGCCCAAT AGAGAAAATT TTTTCTTTCA 540
TCATTATAGT TTGCAAAATG GTATTCCAAA TGTAGTCACA GTAAATAAAA CAATGAAAAA 600
GATGTTAAAT GAGTTAGAAA TTGAGCCAAT AATTACAAGT GAGGTGTTAG ATAAACTTGT 660
GATAGTGTAC TACTAAGTTA AGGTATCGAT AATAAAGTAG TGGCAAAGTT AATGGGCATA 720
AAGATACATC AATGCTAATA AAAATATAAT TAAAAGAATA TGATAAAATA ATAAAAAACC 780
TTGTATATCA ACGATACAAG GTTTTTTATC TATGCCGAGT GCAGGATTGT TTAGATAGTT 840
TTCAAAGTTA AATACAGGCT CTATAAAGCT CTCAAAGTGT TCACTATAGA AAAGCTCTTA 900
AAGTGAAAAA TAAAATGGGG CAAATTGGGG GCATTTCAAG TTAAGAGTAT TCAAATATGT 960
AACTAAAAAA AATTATTTTT TTGAATTAGT GTTTTTCTAT TTGCATATTT TTTCGAGATT 1020
TTCTGTTTTG ATTATTATAT AATTATATTT ATATTTATAG TTATAGTTAT TGAGTTAAGT 1080
TAGTGGGAGA TACA ATG AAA TTA TAT ATA GTT GGT AAT GGC TTT GAT TTA 1130
Met Lys Leu Tyr Ile Val Gly Asn Gly Phe Asp Leu
1               5                   10

TCA CAC AAT CTG AAA ACA TCA TAT ACA GAG TTT AGA TTA TAT TTA TTG 1178
Ser His Asn Leu Lys Thr Ser Tyr Thr Glu Phe Arg Leu Tyr Leu Leu
        15                  20                  25

GAA CAT AGG GAT GAA GTA TAT ACA GAT GAA GAT TTA ATA ATA TCA AAA 1226
Glu His Arg Asp Glu Val Tyr Thr Asp Glu Asp Leu Ile Ile Ser Lys
    30                  35                  40

GGA GAT ATT TTA CGA AAT TTT GAA AGA TAT TGT CAA CCA AAT GAT TTA 1274
Gly Asp Ile Leu Arg Asn Phe Glu Arg Tyr Cys Gln Pro Asn Asp Leu
45                  50                  55                  60

TGG AGC GAT TTT GAA GAA CAG ACT GAG AAA ATA ATA TCA GAA ATA TCA 1322
Trp Ser Asp Phe Glu Glu Gln Thr Glu Lys Ile Ile Ser Glu Ile Ser
                65                  70                  75

GAG GGG AAA ATT ATA ATC TTT GAA GAA GAA TGG AGC TGC AAA TTA GAT 1370
Glu Gly Lys Ile Ile Ile Phe Glu Glu Glu Trp Ser Cys Lys Leu Asp
            80                  85                  90

ATT GGA GGT GAG AGG CAT AAA TTT ATT GAT TGT TTT AAA AAG TTA ATA 1418
Ile Gly Gly Glu Arg His Lys Phe Ile Asp Cys Phe Lys Lys Leu Ile
        95                  100                 105

AAA AAA CAA GAT AGT ACA TTC GAT ACT GAT ATA TAT CAT AAT GAA ATT 1466
Lys Lys Gln Asp Ser Thr Phe Asp Thr Asp Ile Tyr His Asn Glu Ile
    110                 115                 120

CAA GAG TAT GCC TTA AAG TAT TTT AAC AAT AAG GTA AGG GAA CTA TAT 1514
Gln Glu Tyr Ala Leu Lys Tyr Phe Asn Asn Lys Val Arg Glu Leu Tyr
125                 130                 135                 140

ATA TGG GTT CCA TAT TTA TAT ATT AGT TTT CAA GAA TGG ATA CAA ACG 1562
Ile Trp Val Pro Tyr Leu Tyr Ile Ser Phe Gln Glu Trp Ile Gln Thr
                145                 150                 155

ATT ATT TTA AAA AAT ACG AAA AAT ATC TAT GAA ATA GAT GAA GAT TCG 1610
Ile Ile Leu Lys Asn Thr Lys Asn Ile Tyr Glu Ile Asp Glu Asp Ser
            160                 165                 170

TCT GTA ATT TCC TTT AAC TAT ACA AAT ACT ATG GAA GAT GTT TAT CAA 1658
Ser Val Ile Ser Phe Asn Tyr Thr Asn Thr Met Glu Asp Val Tyr Gln
        175                 180                 185

CAA AAA GAT GTA CTA CAT TTA CAT GGT TCT GTA AAA AAT CTA GAA GAT 1706
Gln Lys Asp Val Leu His Leu His Gly Ser Val Lys Asn Leu Glu Asp
    190                 195                 200

GTA GTA TTA GGA TTT CAT TCG CCA GAA ATA GAC GAC AAA TTA CCA GGG 1754
Val Val Leu Gly Phe His Ser Pro Glu Ile Asp Asp Lys Leu Pro Gly
205                 210                 215                 220

TTA GAA ACA AGT TTT ACG AAG GAA TTT AAG GAA ACA CAA AGA ATG TTT 1802
Leu Glu Thr Ser Phe Thr Lys Glu Phe Lys Glu Thr Gln Arg Met Phe
                225                 230                 235

GCA GAA CAG GGT TCT AGA AAG CTT AAT TCT AAT AAG TTT TAT GAT GAA 1850
Ala Glu Gln Gly Ser Arg Lys Leu Asn Ser Asn Lys Phe Tyr Asp Glu
            240                 245                 250

AAT ATA GGA AGA TTT TAT AAA CCA GTA AAT CTT TTA AAA GAC TCA ATT 1898
Asn Ile Gly Arg Phe Tyr Lys Pro Val Asn Leu Leu Lys Asp Ser Ile
        255                 260                 265

GAG TCT TTT GTT AAA AAT AAA AAT ATT CAT GAA GTA ATA ATC TTA GGT 1946
Glu Ser Phe Val Lys Asn Lys Asn Ile His Glu Val Ile Ile Leu Gly
    270                 275                 280

CAT TCA TAT AAT AAG ATA GAC TGG GTT TAT TTT AAA GAA CTA GTC AGA 1994
His Ser Tyr Asn Lys Ile Asp Trp Val Tyr Phe Lys Glu Leu Val Arg
285                 290                 295                 300

TGC GCC CCT GAA GCA AAA TAT TTA TTT AGC TAT TAT TCC CAA AAT GAT 2042
Cys Ala Pro Glu Ala Lys Tyr Leu Phe Ser Tyr Tyr Ser Gln Asn Asp
                305                 310                 315

AAA GAA AAC ATA AAT AAA ATT ATA TTG GAG AAT AAA TTT GAT ATT GAT 2090
Lys Glu Asn Ile Asn Lys Ile Ile Leu Glu Asn Lys Phe Asp Ile Asp
            320                 325                 330

TGC GAA AAA ATA CAT GTA GAT AAC TTT AAA ATT AAA AAA GAT 2132
Cys Glu Lys Ile His Val Asp Asn Phe Lys Ile Lys Lys Asp
        335                 340                 345

TAGCAAAAAT TTAGGAGTTT TATATGATTA TATTTATAGA CCCGAATTGC TAGTTGATTA 2192
TTTAGCCATG ACTTGATACC CGATAGAATA TCTTAAAGTC TCTGGTTCCA GTGATTTAGC 2252
TGATTTTAAC AGTAAAGAAT ACGCTAAAAG TATCATCTCT AATTTCAATT GAAAACCTTG 2312
AGGCGAACGA CTTTTACAAC GCTCAGCTCC TAGATTTGTC AAAAAAGAGA AAACTCGCTC 2372
AATCACTTTT CTACGTTTTG AAAAATTAGG GAAAAGGATT TTCTTTCGCT TCATGTTCTT 2432
CCTGACAGGT GTCATTAGAT CAATTCCTTT TAATTCCAGC CTATCATGCA GTGACTGACC 2492
TAAATATCCC ATATCTCCAA GGACTGTTGG TGTCCCAAAT TGACTCAACA CTTCCTCGGC 2552
CATTGTACTG TCAGCTATTG AAGCAGGAGT AATTGTGTAG TCTATGACAT AGCCTGATTC 2612
ACTGACTAAA GCATGACATT TACATCCATA GAAGTACTGT CCCTTTGTAA CATTGTAGCC 2672
AACATTTGCA TAATCTCCAA GAACTTTGCT TCTGAAATTA CGAATCGACT GACACAAAGG 2732
AATGGGGAAG CTGTCAATAA TGGATACACT CATTCCTTCA ACCTCTTTAA AGACGAGTGC 2792
TTGGCGAATG ACTTGGATAC TCGGTAAGAG GGCATTACAA CGGCGGACAA AGCGAGAATA 2852
TTCTAGGAAA TTAGGAAATA AACTTTGAGC CAATTGGTGC TTAGCTTTAA GTGTTTCACT 2912
AAAATGCAGT ACGCCCCATA GGTAACAAGC GATAACTAAG CAATCTGATG TTGCGAGATG 2972

GACGTTCTTT CGGTTTTGAA CCTCAAGGGG ACACTCGTTT GATAAATCGT CTCAATGGTT 3032
GTCAGTAAAT AAACCAAAAA CTTTTGGAAG TGTGCTATTA TAAGTCATAT AAGTCGTGCG 3092
CTTTCTAATG CTTAGTGGGT TAAGATTAGG ATAGCACGAC TTATTTATTT TCCAATGAAA 3152
TTAACTAGCA ATTCGGGTTT TCTTAATTTT TTTTATAAAA AAATTACTCC TAAAGCAGAG 3212
TAATTTTATT GTTAATTTTT ATCAGAGATT ATTCCAAAAA TTTTTCTGAT AAAAATGAAG 3272
TATCACTCCT ACCGAAAATC CTAAAAGGAG GACTAGGGCA AGGTTAACAA CTAAAGGATA 3332
TGGAATAAAG ATAATCAGGG CAATAATTGC TCCAATGAGT GCTCCTGCTC CCAAAAGCGT 3392
AGCAAAGAAA ATGATAAAAA CCCGAAATCC CCCAGAACCT CTTGTAAAAA GTTGAGTCAA 3452
CGCAGTCCAA TTTAAATTTA AGAGATGCCA ATCTCTGACA AAATAATGAA GTGAAATCAG 3512
ATATACCCCA ATAACATTCC CAATTAACAG CCCAAAGAGC AAGGTAACAG GGACTTTCAG 3572
TAAAATACCA ATCACTAAAG ATAAGATTAA GTCAAGAACT AGTTGAATAA AAAAGGCAAA 3632
ATGAAATTTC ACTCTGAGAT AGAAATTCAA CTTCATGGGT AAGCTTTTAA AATAATTAAA 3692
ATTTTCCCGA TC 3704

(2) INFORMATION POUR LA SEQ ID NO: 2:

(i) CARACTERISTIQUES DE LA SEQUENCE:
(A) LONGUEUR: 346 acides aminés
(B) TYPE: acide aminé
(D) CONFIGURATION: linéaire

(ii) TYPE DE MOLECULE: protéine

(xi) DESCRIPTION DE LA SEQUENCE: SEQ ID NO: 2:

Met Lys Leu Tyr Ile Val Gly Asn Gly Phe Asp Leu Ser His Asn Leu
1               5                   10                  15
Lys Thr Ser Tyr Thr Glu Phe Arg Leu Tyr Leu Leu Glu His Arg Asp
            20                  25                  30
Glu Val Tyr Thr Asp Glu Asp Leu Ile Ile Ser Lys Gly Asp Ile Leu
        35                  40                  45
Arg Asn Phe Glu Arg Tyr Cys Gln Pro Asn Asp Leu Trp Ser Asp Phe
    50                  55                  60
Glu Glu Gln Thr Glu Lys Ile Ile Ser Glu Ile Ser Glu Gly Lys Ile
65                  70                  75                  80
Ile Ile Phe Glu Glu Glu Trp Ser Cys Lys Leu Asp Ile Gly Gly Glu
                85                  90                  95
Arg His Lys Phe Ile Asp Cys Phe Lys Lys Leu Ile Lys Lys Gln Asp
            100                 105                 110
Ser Thr Phe Asp Thr Asp Ile Tyr His Asn Glu Ile Gln Glu Tyr Ala
        115                 120                 125
Leu Lys Tyr Phe Asn Asn Lys Val Arg Glu Leu Tyr Ile Trp Val Pro
    130                 135                 140

Tyr Leu Tyr Ile Ser Phe Gln Glu Trp Ile Gln Thr Ile Ile Leu Lys
145                 150                 155                 160
Asn Thr Lys Asn Ile Tyr Glu Ile Asp Glu Asp Ser Ser Val Ile Ser
                165                 170                 175
Phe Asn Tyr Thr Asn Thr Met Glu Asp Val Tyr Gln Gln Lys Asp Val
            180                 185                 190
Leu His Leu His Gly Ser Val Lys Asn Leu Glu Asp Val Val Leu Gly
        195                 200                 205
Phe His Ser Pro Glu Ile Asp Asp Lys Leu Pro Gly Leu Glu Thr Ser
    210                 215                 220
Phe Thr Lys Glu Phe Lys Glu Thr Gln Arg Met Phe Ala Glu Gln Gly
225                 230                 235                 240
Ser Arg Lys Leu Asn Ser Asn Lys Phe Tyr Asp Glu Asn Ile Gly Arg
                245                 250                 255
Phe Tyr Lys Pro Val Asn Leu Leu Lys Asp Ser Ile Glu Ser Phe Val
            260                 265                 270
Lys Asn Lys Asn Ile His Glu Val Ile Ile Leu Gly His Ser Tyr Asn
        275                 280                 285
Lys Ile Asp Trp Val Tyr Phe Lys Glu Leu Val Arg Cys Ala Pro Glu
    290                 295                 300
Ala Lys Tyr Leu Phe Ser Tyr Tyr Ser Gln Asn Asp Lys Glu Asn Ile
305                 310                 315                 320
Asn Lys Ile Ile Leu Glu Asn Lys Phe Asp Ile Asp Cys Glu Lys Ile
                325                 330                 335
His Val Asp Asn Phe Lys Ile Lys Lys Asp
            340                 345

(2) INFORMATION POUR LA SEQ ID NO: 3:

     (i) CARACTERISTIQUES DE LA SEQUENCE:
          (A) LONGUEUR: 1345 paires de bases
          (B) TYPE: acide nucléique
          (C) NOMBRE DE BRINS: simple
          (D) CONFIGURATION: linéaire

    (ii) TYPE DE MOLECULE: ADN (génomique)

   (iii) HYPOTHETIQUE: NON

   (iii) ANTI-SENS: NON

    (vi) ORIGINE
          (A) ORGANISME: Lactococcus lactis

(ix) CARACTERISTIQUE ADDITIONELLE:
(A) NOM/CLE: CDS
(B) EMPLACEMENT: 246..1283

(xi) DESCRIPTION DE LA SEQUENCE: SEQ ID NO: 3:

AAATACAGGC TCTATAAAGC TCTCAAAGTG TTCACTATAG AAAAGCTCTT AAAGTGAAAA 60
ATAAAATGGG GCAAATTGGG GGCATTTCAA GTTAAGAGTA TTCAAATATG TAACTAAAAA 120
AAATTATTTT TTTGAATTAG TGTTTTTCTA TTTGCATATT TTTTCGAGAT TTTCTGTTTT 180
GATTATTATA TAATTATATT TATATTTATA GTTATAGTTA TTGAGTTAAG TTAGTGGGAG 240
ATACA ATG AAA TTA TAT ATA GTT GGT AAT GGC TTT GAT TTA TCA CAC 287
Met Lys Leu Tyr Ile Val Gly Asn Gly Phe Asp Leu Ser His
1               5                   10

AAT CTG AAA ACA TCA TAT ACA GAG TTT AGA TTA TAT TTA TTG GAA CAT 335
Asn Leu Lys Thr Ser Tyr Thr Glu Phe Arg Leu Tyr Leu Leu Glu His
15                  20                  25                  30

AGG GAT GAA GTA TAT ACA GAT GAA GAT TTA ATA ATA TCA AAA GGA GAT 383
Arg Asp Glu Val Tyr Thr Asp Glu Asp Leu Ile Ile Ser Lys Gly Asp
                35                  40                  45

ATT TTA CGA AAT TTT GAA AGA TAT TGT CAA CCA AAT GAT TTA TGG AGC 431
Ile Leu Arg Asn Phe Glu Arg Tyr Cys Gln Pro Asn Asp Leu Trp Ser
            50                  55                  60

GAT TTT GAA GAA CAG ACT GAG AAA ATA ATA TCA GAA ATA TCA GAG GGG 479
Asp Phe Glu Glu Gln Thr Glu Lys Ile Ile Ser Glu Ile Ser Glu Gly
        65                  70                  75

AAA ATT ATA ATC TTT GAA GAA GAA TGG AGC TGC AAA TTA GAT ATT GGA 527
Lys Ile Ile Ile Phe Glu Glu Glu Trp Ser Cys Lys Leu Asp Ile Gly
    80                  85                  90

GGT GAG AGG CAT AAA TTT ATT GAT TGT TTT AAA AAG TTA ATA AAA AAA 575
Gly Glu Arg His Lys Phe Ile Asp Cys Phe Lys Lys Leu Ile Lys Lys
95                  100                 105                 110

CAA GAT AGT ACA TTC GAT ACT GAT ATA TAT CAT AAT GAA ATT CAA GAG 623
Gln Asp Ser Thr Phe Asp Thr Asp Ile Tyr His Asn Glu Ile Gln Glu
                115                 120                 125

TAT GCC TTA AAG TAT TTT AAC AAT AAG GTA AGG GAA CTA TAT ATA TGG 671
Tyr Ala Leu Lys Tyr Phe Asn Asn Lys Val Arg Glu Leu Tyr Ile Trp
            130                 135                 140

GTT CCA TAT TTA TAT ATT AGT TTT CAA GAA TGG ATA CAA ACG ATT ATT 719
Val Pro Tyr Leu Tyr Ile Ser Phe Gln Glu Trp Ile Gln Thr Ile Ile
        145                 150                 155

TTA AAA AAT ACG AAA AAT ATC TAT GAA ATA GAT GAA GAT TCG TCT GTA 767
Leu Lys Asn Thr Lys Asn Ile Tyr Glu Ile Asp Glu Asp Ser Ser Val
    160                 165                 170

ATT TCC TTT AAC TAT ACA AAT ACT ATG GAA GAT GTT TAT CAA CAA AAA 815
Ile Ser Phe Asn Tyr Thr Asn Thr Met Glu Asp Val Tyr Gln Gln Lys
175                 180                 185                 190

GAT GTA CTA CAT TTA CAT GGT TCT GTA AAA AAT CTA GAA GAT GTA GTA 863
Asp Val Leu His Leu His Gly Ser Val Lys Asn Leu Glu Asp Val Val
                195                 200                 205

TTA GGA TTT CAT TCG CCA GAA ATA GAC GAC AAA TTA CCA GGG TTA GAA 911
Leu Gly Phe His Ser Pro Glu Ile Asp Asp Lys Leu Pro Gly Leu Glu
            210                 215                 220

ACA AGT TTT ACG AAG GAA TTT AAG GAA ACA CAA AGA ATG TTT GCA GAA 959
Thr Ser Phe Thr Lys Glu Phe Lys Glu Thr Gln Arg Met Phe Ala Glu
        225                 230                 235

CAG GGT TCT AGA AAG CTT AAT TCT AAT AAG TTT TAT GAT GAA AAT ATA 1007
Gln Gly Ser Arg Lys Leu Asn Ser Asn Lys Phe Tyr Asp Glu Asn Ile
    240                 245                 250

GGA AGA TTT TAT AAA CCA GTA AAT CTT TTA AAA GAC TCA ATT GAG TCT 1055
Gly Arg Phe Tyr Lys Pro Val Asn Leu Leu Lys Asp Ser Ile Glu Ser
255                 260                 265                 270

TTT GTT AAA AAT AAA AAT ATT CAT GAA GTA ATA ATC TTA GGT CAT TCA 1103
Phe Val Lys Asn Lys Asn Ile His Glu Val Ile Ile Leu Gly His Ser
                275                 280                 285

TAT AAT AAG ATA GAC TGG GTT TAT TTT AAA GAA CTA GTC AGA TGC GCC 1151
Tyr Asn Lys Ile Asp Trp Val Tyr Phe Lys Glu Leu Val Arg Cys Ala
            290                 295                 300

CCT GAA GCA AAA TAT TTA TTT AGC TAT TAT TCC CAA AAT GAT AAA GAA 1199
Pro Glu Ala Lys Tyr Leu Phe Ser Tyr Tyr Ser Gln Asn Asp Lys Glu
        305                 310                 315

AAC ATA AAT AAA ATT ATA TTG GAG AAT AAA TTT GAT ATT GAT TGC GAA 1247
Asn Ile Asn Lys Ile Ile Leu Glu Asn Lys Phe Asp Ile Asp Cys Glu
    320                 325                 330

AAA ATA CAT GTA GAT AAC TTT AAA ATT AAA AAA GAT TAGCAAAAAT 1293
Lys Ile His Val Asp Asn Phe Lys Ile Lys Lys Asp
335                 340                 345

TTAGGAGTTT TATATGATTA TATTTATAGA CCCGAATTGC TAGTTGATTA TT 1345

(2) INFORMATION POUR LA SEQ ID NO: 4:

(i) CARACTERISTIQUES DE LA SEQUENCE:
(A) LONGUEUR: 346 acides aminés
(B) TYPE: acide aminé
(D) CONFIGURATION: linéaire

(ii) TYPE DE MOLECULE: protéine

(xi) DESCRIPTION DE LA SEQUENCE: SEQ ID NO: 4:

Met Lys Leu Tyr Ile Val Gly Asn Gly Phe Asp Leu Ser His Asn Leu
1               5                   10                  15
Lys Thr Ser Tyr Thr Glu Phe Arg Leu Tyr Leu Leu Glu His Arg Asp
            20                  25                  30
Glu Val Tyr Thr Asp Glu Asp Leu Ile Ile Ser Lys Gly Asp Ile Leu
        35                  40                  45
Arg Asn Phe Glu Arg Tyr Cys Gln Pro Asn Asp Leu Trp Ser Asp Phe
    50                  55                  60
Glu Glu Gln Thr Glu Lys Ile Ile Ser Glu Ile Ser Glu Gly Lys Ile
65                  70                  75                  80
Ile Ile Phe Glu Glu Glu Trp Ser Cys Lys Leu Asp Ile Gly Gly Glu
                85                  90                  95
Arg His Lys Phe Ile Asp Cys Phe Lys Lys Leu Ile Lys Lys Gln Asp
            100                 105                 110
Ser Thr Phe Asp Thr Asp Ile Tyr His Asn Glu Ile Gln Glu Tyr Ala
        115                 120                 125

Leu Lys Tyr Phe Asn Asn Lys Val Arg Glu Leu Tyr Ile Trp Val Pro
    130                 135                 140
Tyr Leu Tyr Ile Ser Phe Gln Glu Trp Ile Gln Thr Ile Ile Leu Lys
145                 150                 155                 160
Asn Thr Lys Asn Ile Tyr Glu Ile Asp Glu Asp Ser Ser Val Ile Ser
                165                 170                 175
Phe Asn Tyr Thr Asn Thr Met Glu Asp Val Tyr Gln Gln Lys Asp Val
            180                 185                 190
Leu His Leu His Gly Ser Val Lys Asn Leu Glu Asp Val Val Leu Gly
        195                 200                 205
Phe His Ser Pro Glu Ile Asp Asp Lys Leu Pro Gly Leu Glu Thr Ser
    210                 215                 220
Phe Thr Lys Glu Phe Lys Glu Thr Gln Arg Met Phe Ala Glu Gln Gly
225                 230                 235                 240
Ser Arg Lys Leu Asn Ser Asn Lys Phe Tyr Asp Glu Asn Ile Gly Arg
                245                 250                 255
Phe Tyr Lys Pro Val Asn Leu Leu Lys Asp Ser Ile Glu Ser Phe Val
            260                 265                 270
Lys Asn Lys Asn Ile His Glu Val Ile Ile Leu Gly His Ser Tyr Asn
        275                 280                 285
Lys Ile Asp Trp Val Tyr Phe Lys Glu Leu Val Arg Cys Ala Pro Glu
    290                 295                 300
Ala Lys Tyr Leu Phe Ser Tyr Tyr Ser Gln Asn Asp Lys Glu Asn Ile
305                 310                 315                 320
Asn Lys Ile Ile Leu Glu Asn Lys Phe Asp Ile Asp Cys Glu Lys Ile
                325                 330                 335
His Val Asp Asn Phe Lys Ile Lys Lys Asp
            340                 345

(2) INFORMATION POUR LA SEQ ID NO: 5:

     (i) CARACTERISTIQUES DE LA SEQUENCE:
          (A) LONGUEUR: 31 paires de bases
          (B) TYPE: acide nucléique
          (C) NOMBRE DE BRINS: simple
          (D) CONFIGURATION: linéaire

    (ii) TYPE DE MOLECULE: ADN (génomique)

   (iii) HYPOTHETIQUE: NON

   (iii) ANTI-SENS: NON

(ix) CARACTERISTIQUE ADDITIONELLE:
(A) NOM/CLE: misc_signal
(B) EMPLACEMENT: 6..11
(D) AUTRES RENSEIGNEMENTS: /function= "site de restriction
BamHI"

(ix) CARACTERISTIQUE ADDITIONELLE:
(A) NOM/CLE: misc_structure
(B) EMPLACEMENT: 12..31
(D) AUTRES RENSEIGNEMENTS: /function= "séquence homologue à
l'ADNc correspondant aux nucléotides 1325-1344 de
SEQ.ID NO: 3"

(xi) DESCRIPTION DE LA SEQUENCE: SEQ ID NO: 5:

TCATAGGATC CAATAATCAA CTAGCAATTC G 31

(2) INFORMATION POUR LA SEQ ID NO: 6:

(i) CARACTERISTIQUES DE LA SEQUENCE:
(A) LONGUEUR: 31 paires de bases
(B) TYPE: acide nucléique
(C) NOMBRE DE BRINS: simple
(D) CONFIGURATION: linéaire

(ii) TYPE DE MOLECULE: ADN (génomique)

(iii) HYPOTHETIQUE: NON

(iii) ANTI-SENS: NON

(ix) CARACTERISTIQUE ADDITIONELLE:
(A) NOM/CLE: misc_signal
(B) EMPLACEMENT: 6..11
(D) AUTRES RENSEIGNEMENTS: /function= "site de restriction
SalI"

(ix) CARACTERISTIQUE ADDITIONELLE:
(A) NOM/CLE: misc_structure
(B) EMPLACEMENT: 12..31
(D) AUTRES RENSEIGNEMENTS: /function= "séquence homologue
aux nucléotides 1-20 de SEQ. ID NO:3"

(xi) DESCRIPTION DE LA SEQUENCE: SEQ ID NO: 6:

CATTAGTCGA CAAATACAGG CTCTATAAAG C 31

(2) INFORMATION POUR LA SEQ ID NO: 7:

(i) CARACTERISTIQUES DE LA SEQUENCE:
(A) LONGUEUR: 32 paires de bases
(B) TYPE: acide nucléique
(C) NOMBRE DE BRINS: simple
(D) CONFIGURATION: linéaire

(ii) TYPE DE MOLECULE: ADN (génomique)

(iii) HYPOTHETIQUE: NON

(iii) ANTI-SENS: NON

(ix) CARACTERISTIQUE ADDITIONELLE:
(A) NOM/CLE: misc_signal
(B) EMPLACEMENT: 6..11
(D) AUTRES RENSEIGNEMENTS: /function= "site de restriction
BamHI"

(xi) DESCRIPTION DE LA SEQUENCE: SEQ ID NO: 7:

TCATAGGATC CTTAACAATA AAATTACTCT GC 32

(2) INFORMATION POUR LA SEQ ID NO: 8:

(i) CARACTERISTIQUES DE LA SEQUENCE:
(A) LONGUEUR: 32 paires de bases
(B) TYPE: acide nucléique
(C) NOMBRE DE BRINS: simple
(D) CONFIGURATION: linéaire

(ii) TYPE DE MOLECULE: ADN (génomique)

(iii) HYPOTHETIQUE: NON

(iii) ANTI-SENS: NON

(ix) CARACTERISTIQUE ADDITIONELLE:

(A) NOM/CLE: misc_signal
(B) EMPLACEMENT: 6..11
(D) AUTRES RENSEIGNEMENTS: /function= "site de restriction
SalI"

(xi) DESCRIPTION DE LA SEQUENCE: SEQ ID NO: 8:

CATTAGTCGA CTTAAATTTG ATATTGATTG CG 32

## Description

La présente invention a pour objet des nouvelles séquences d'acides nucléiques et des plasmides susceptibles de s'hybrider avec celles-ci, porteurs d'au moins un mécanisme de résistance aux phages, les bactéries lactiques contenant ces séquences ou ces plasmides, en particulier les lactocoques appartenant à l'espèce Lactococcus lactis, l'utilisation de certaines souches de ces lactocoques pour transférer, notamment par conjugaison, un mécanisme de résistance aux phages à des souches d'intérêt industriel, en particulier dans l'industrie laitière, et à l'utilisation de certaines souches de Lactococcus lactis pour obtenir ces plasmides.

Les bactéries lactiques sont impliquées dans l'élaboration et la conservation d'un grand nombre de produits alimentaires, tels que les fromages, le beurre, les yaourts, le saucisson ou la choucroute. Parmi ceux-ci les produits laitiers occupent une place particulièrement importante. La transformation industrielle du lait est faite dans des cuves de fermentation de plus en plus grandes, dans lesquelles l'apparition de phages des bactéries lactiques peut avoir des conséquences graves, voire catastrophiques : variation des caractéristiques, notamment organoleptiques, du produit final ; perte du produit présent dans la cuve et nécessité de décontaminer cette dernière ainsi que les installations environnantes. Il existe donc dans l'industrie laitière un besoin impérieux de nouveaux moyens et de nouvelles méthodes permettant de rendre les bactéries plus résistantes aux phages.

Les phages des bactéries lactiques appartiennent à trois grands groupes d'homologie (I), (II) et (III) définis par des études d'hybridation ADN/ADN selon RELANO P. et al (1987), J. Gen. Microbiol. 133, 3053-3063. Les groupes (I) et (III) comprennent uniquement des phages virulents. Le groupe (II) comprend des phages virulents et des phages tempérés. A l'intérieur d'un même groupe les homologies sont fortes et, d'un groupe à l'autre, les homologies sont très faibles. Les phages du groupe (I) ont une nucléocapside oblongue alors que les phages des groupes (II) et (III) ont une nucléocapside isométrique.

On sait qu'il existe plusieurs mécanismes naturels de résistance aux phages dont les trois principaux sont :
- l'inhibition de l'adsorption des phages ; dans ce mécanisme, l'adsorption du phage par la bactérie est inhibée ou retardée.
- le système restriction/modification ; ce système fait intervenir une enzyme de restriction qui dégrade l'ADN du phage dès son entrée dans la bactérie.
- l'infection abortive ; selon ce troisième mécanisme, l'adsorption des phages est normale, mais leur multiplication ne se produit pas.

Ces mécanismes sont décrits en détail par SANDERS M. dans Biochimie 70, (1988), 411-421.

De nombreuses études ont déjà été effectuées pour mettre au point des bactéries lactiques résistantes aux phages.

A cet effet, on peut se référer en particulier aux articles ci-après :
- VLEGELS et al. ; Neth. Milk and Dairy J. 43 (1989) 245-259
- SANDERS and KLAENHAMMER. Applied and Environ. Microbiol. (1983) vol. 46, 1125-1133
qui concernent des plasmides qui inhibent l'adsorption des phages ;
- Audrey W. JARVIS ; Applied and Environ. Microbiol. ; March 1988 p. 777-783 ;
- EP-A₃-0208 468 ;
- COFFEY et al. ; Neth. Milk and Dairy J. 43 (1989) 229-244 ;
- KLAENHAMMER and SANOZKY ; Journal of General Microbiology (1985), 131, 1531-1541
- DURMAZ et al.; J. Bacteriol. (1992) 7463-7469
qui décrivent des plasmides qui confèrent une résistance aux phages par le mécanisme d'infection abortive.
- JOSEPHSEN and KLAENHAMMER, Plasmid 23, 71-75 (1990)
- MOINEAU et al.; Applied and Environ. Microbiol. (1995) 2193-2202
- brevet US 4 883 756
- GAUTIER and CHOPIN ; Applied and Environ. Microbiol. (1987) 53 p. 923-927,
- McLANDSBOROUGH et al.; Applied and Environ. Microbiol. (1995) 2023-2026
ces derniers articles décrivent notamment des plasmides conférant la résistance aux phages par le mécanisme de restriction/modification.

Dans la demande EP-A1-452 224 une séquence d'ADN comprenant au moins un mécanisme de résistance aux phages est également décrite ; cette séquence d'ADN comporte une partie fonctionnelle du fragment HindIII-HindIII d'environ 3,3 kb du plasmide pPF 144-1 présent dans la souche Escherichia coli déposée le 9 avril 1991 à la C.N.C.M. (Collection Nationale de Cultures de Microorganismes, Institut Pasteur, Paris, France) sous le N°1-1070.

Ce fragment HindIII-HindIII d'environ 3,3 kb, a été isolé à partir du plasmide pPF144 contenu dans la souche Lactococcus lactis ssp lactis, déposée à la C.N.C.M. le 12 avril 1990 sous le N°I-945, laquelle est un transconjugant issu du croisement de la souche donneuse Lactococcus lactis ssp lactis S91 déposée à la C.N.C.M. le 12 Avril 1990 sous le N°I-940 et de la souche réceptrice Lactococcus lactis ssp lactis S45, dérivée de la souche Lactococcus lactis ssp lactis C2-LL. décrite par Mc. Kay et al, 1977, dans J. Bacteriol., 257-265. Ce fragment est porteur d'un ou plusieurs mécanismes de résistance aux phages.

A la suite de ces travaux, une séquence d'ADN de 1,9 kb qui confère à elle seule la résistance aux phages a été isolée à partir de cette séquence d'ADN HindIII-HindIII de 3,3 kb. Cette nouvelle séquence d'ADN d'environ 1,9 kb a été décrite dans EP-A₁-643134.

La demanderesse a isolé un fragment de 3704 paires de bases (pb) par une digestion partielle à l'aide de l'enzyme de restriction Sau3A de l'ADN total de la souche S94 de Lactococcus lactis ssp diacetylactis déposée à la C.N.C.M. le 12 Avril 1990 sous le N° I-941.

Ce fragment est porteur d'un ou plusieurs mécanismes de résistance aux phages et confère à lui seul la résistance aux phages.

Par la suite, à partir de cette séquence d'ADN de 3704 pb, la demanderesse a isolé une séquence d'ADN de 1345 pb qui confère à elle seule la résistance aux phages.

La présente invention a donc pour objet deux nouvelles séquences d'acides nucléiques comprenant au moins un mécanisme de résistance aux phages, lesdites séquences ayant respectivement 3704 pb et 1345 pb et étant constituées par :
a) les séquences d'ADN présentant l'enchainement d'acides nucléiques [SEQ ID No. 1] ou [SEQ ID No. 3] ;
b) les séquences d'ADN hybridant avec les séquences ci-dessus ou un fragment de celles-ci ;
c) les séquences d'ARNm et d'ADNc correspondantes.

Les séquences [SEQ ID N° 2] et [SEQ ID N° 4] sont les séquences d'acides aminés déduites respectivement des séquences [SEQ ID N° 1] et [SEQ ID N°3].

La séquence [SEQ ID N°3] est un fragment d'ADN de 1345 pb compris dans la séquence [SEQ ID N° 1].

Ce fragment de 1345 pb peut être obtenu par la méthode PCR à l'aide des deux oligonucléotides ci-après :

Oligonucléotide N° 1 [SEQ N° 5] :
5' TCATAGGATCCAATAATCAACTAGCAATTCG 3'
BamHI

Oligonucléotide N° 2 [SEQ N° 6] :
5' CATTAGTCGACAAATACAGGCTCTATAAAGC 3'
SalI

L'invention a plus particulièrement pour objet les séquences d'ADN comprenant au moins un mécanisme de résistance aux phages, lesdites séquences ayant respectivement :
- 3704 pb et présentant l'enchaînement d'acides nucléiques ci-après [SEQ ID N° 1];
- 1345 pb et présentant l'enchaînement d'acides nucléiques ci-après [SEQ ID N°3] ;

L'invention a également pour objet les séquences d'ADN qui présentent un degré d'homologie élevé avec les séquences d'ADN ci-dessus [SEQ ID N°1] et [SEQ ID N°3]. Un degré d'homologie élevé signifie une homologie (rapport entre les nucléotides identiques et le nombre total de nucléotides) d'au moins 70 %, et de préférence d'au moins 80 %, des séquences de nucléotides, lorsqu'elles sont alignées d'après l'homologie maximale, selon la méthode d'alignement optimal des séquences de Needleman et Wunsch, 1970 ; J. Mol. Biol., 48, 443-453. Cette méthode est notamment utilisée dans le logiciel UWGCG de l'Université du Wisconsin : Devereux et al., 1984, Nucl. Ac. Res., 12, 8711-8721 - option GAP.

La présente invention a plus particulièrement pour objet les séquences d'ADN qui s'hybrident avec les séquences d'ADN [SEQ ID No. 1] et [SEQ ID No 3] ou un fragment de celles-ci. Dans la présente description le terme "hybridation" désigne les conditions classiques d'hybridation et plus particulièrement les conditions d'hybridation strictes.

L'invention a également pour objet les plasmides transformés avec l'une des séquences d'acides nucléiques selon l'invention. Ces plasmides peuvent être par exemple le plasmide pLDP1 dans lequel on a cloné, selon les techniques habituelles bien connues de l'homme de l'art, l'une des séquences d'ADN selon l'invention. Le plasmide pLDP1 dérive du plasmide pVA838 (Macrina F.L et al, Gene 19, 345-353) par délétion du fragment de 1523 pb compris entre le site HindIII (0) et le site EcoRI (1523) et remplacement de celui-ci par 54 paires de bases correspondant aux sites multiples de clonage du plasmide pUC18 (Yanisch - Perron C. et al: 1985 - Gene 33, 103-119) bordés par les sites EcoRI et HindIII.

L'invention a également trait aux bactéries lactiques résistantes aux phages, de préférence appartenant à l'espèce Lactococcus lactis. qui contiennent au moins une des séquences d'acides nucléiques ou un plasmide tels que définis ci-dessus.

Ces séquences d'acides nucléiques ou ces plasmides peuvent avoir été introduits dans les bactéries lactiques par conjugaison, transformation, fusion de protoplastes ou par toute autre méthode de transfert de gènes bien connue de l'homme du métier.

Les bactéries lactiques qui peuvent avantageusement être transformées à l'aide des séquences d'ADN selon l'invention ou d'un plasmide les contenant sont par exemple les souches Lactococcus lactis ssp cremoris, Lactococcus lactis ssp lactis, Lactococcus lactis ssp lactis var, diacetylactis.

Ces souches ainsi transformées peuvent être utilisées pour transmettre par conjugaison, transformation, transduction, fusion de protoplastes ou toute autre méthode de transfert de gènes bien connue de l'homme du métier, un mécanisme de résistance aux phages à une souche d'intérêt industriel. Ce mécanisme peut être porté par un plasmide ou par une autre partie du génome de la bactérie. Lorsque celui-là est porté par un plasmide il est avantageux de le transférer par conjugaison.

L'invention a également trait aux souches d'intérêt industriel résistantes aux phages ainsi obtenues.

L'invention sera mieux comprise à l'aide des exemples ci-après, qui comprennent des résultats expérimentaux et une discussion de ceux-ci. Certains de ces exemples concernent des expériences effectuées dans le but de réaliser l'invention, d'autres concernant des exemples de réalisation de l'invention donnés bien sûr à titre purement illustratif.

Une grande partie de l'ensemble des techniques décrites dans ces exemples, bien connues de l'homme de l'art, est exposée en détail dans l'ouvrage de Sambrook Fritsch et Maniatis: "Molecular cloning ; a Laboratory Manual" publié en 1989 par les éditions Cold Spring Harbor Press à New York (2e édition).

La description ci-après sera mieux comprise à l'aide des figures 1 à 2 ci-après qui représentent respectivement :
- FIG. 1 :: Organisation des ORF déduites de la séquence du fragment de 3704 paires de bases.
- FIG. 2 :: Amplification par PCR de 2 fragments internes au fragment de 3704 pb conférant la résistance aux phages.

### Exemple 1 : Séquence du fragment de 3704 pb

La souche S94 de Lactococcus lactis ssp diacetylactis déposée à la C.N.C.M. le 13 Avril 1990 sous le N° I-941, contient un ou plusieurs mécanismes de résistance aux phages. En particulier, elle a transféré par conjugaison dans la souche réceptrice Lactococcus lactis S45, dérivée de la souche Lactococcus lactis C2-LL décrite par McKay et al., 1977, J. Bacteriol. 257-265, le plasmide pPF72 qui confère la résistance aux phages Ø 53 (groupe I) et Ø 59 (groupe III). D'autres mécanismes de résistance aux phages peuvent être présents dans la souche I-941. C'est pourquoi une banque d'ADN total a été construite à partir de la souche I-941. Le fragment de restriction de 3704 pb conférant la résistance aux phages, obtenu par une digestion partielle à l'aide de l'enzyme de restriction Sau3A de l'ADN total de la souche S94 (I-941) a été cloné dans le vecteur pLDP1 au site BamHI. Le plasmide recombinant pLAB206, obtenu, confère à la souche Lactococcus lactis ssp lactis MG 1363 décrite par GASSON M.J. (1983) dans J. Bacteril. 154 : 1-9, dénommée ci-après souche L.lactis S56 ou S56, une résistance totale au phage Ø 59.

La séquence d'acides nucléiques de ce fragment de 3704 pb déterminée par la méthode de Sanger et al. (PNAS-USA, 14, 5463, 1977) est la séquence [SEQ IDN° 1] ci-après.

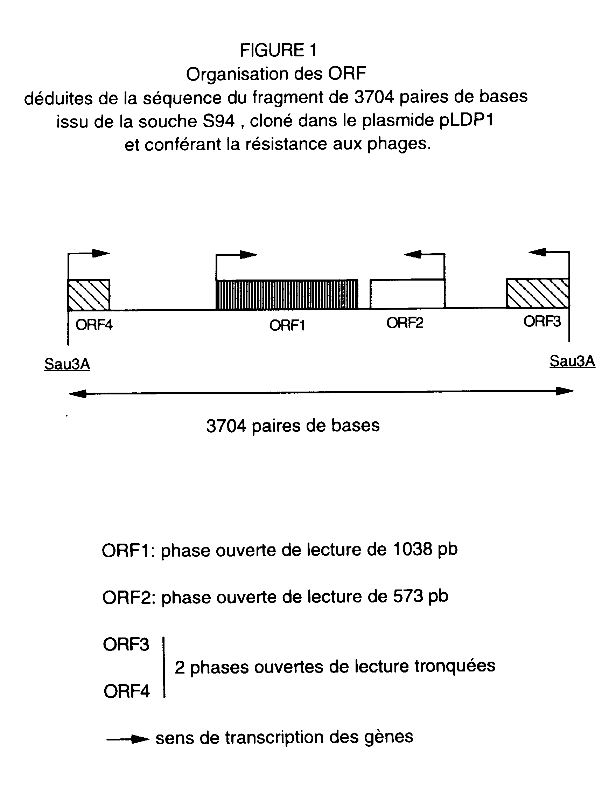

L'analyse de la séquence a montré que le fragment de 3704 pb possède 2 phases de lecture ouvertes complètes (ORF1 et ORF2) et deux phases de lecture ouvertes tronquées (ORF3 et ORF4) ne comportant que les parties N-terminales (figure 1).

### Exemple 2 : Amplification par PCR d'un fragment interne du fragment de 3704 pb

La technique de la PCR (Polymerase Chain Reaction), décrite par exemple dans l'ouvrage de Maniatis, déjà cité, permet d'amplifier un fragment d'ADN compris entre deux oligonucléotides convenablement choisis. Cet ADN amplifié peut être aisément clone si des sites de restriction sont apportés par les oligonucléotides. En effet, les séquences de ces oligonucléotides peuvent comporter, à leur extrémité 5', une partie hétérologue de l'ADN à amplifier, constituée par exemple de 10 à 12 paires de bases, dont 6 constituent un site de restriction.

Cette technique a été appliquée en vue de déterminer si l'un ou l'autre des deux cadres ouverts de lecture (ORF1, ORF2) mis en évidence dans la séquence nucléotidique du fragment de 3704 pb correspond à un gène de résistance aux phages, mais également en vue de constituer une sonde spécifique de ce gène.

Pour cela, 4 oligonucléotides de 31 et 32 bases (dont 6 constituent un site de restriction) ont été synthétisés.

La séquence de ces 4 oligonucléotides est la suivante :

Oligonucléotide N° 1 [SEQ N° 5] :
5' TCATAGGATCCAATAATCAACTAGCAATTCG 3'
BamHI

Oligonucléotide N° 2 [SEQ N°6] :
5' CATTAGTCGACAAATACAGGCTCTATAAAGC 3'
SalI

Oligonucléotide N° 3 [SEQ N°7] :
5' TCATAGGATCCTTAACAATAAAATTACTCTGC 3'
BamHI

Oligonucléotide N° 4 [SEQ N° 8]
5' CATTAGTCGACTTAAATTTGATATTGATTGCG 3'
SalI

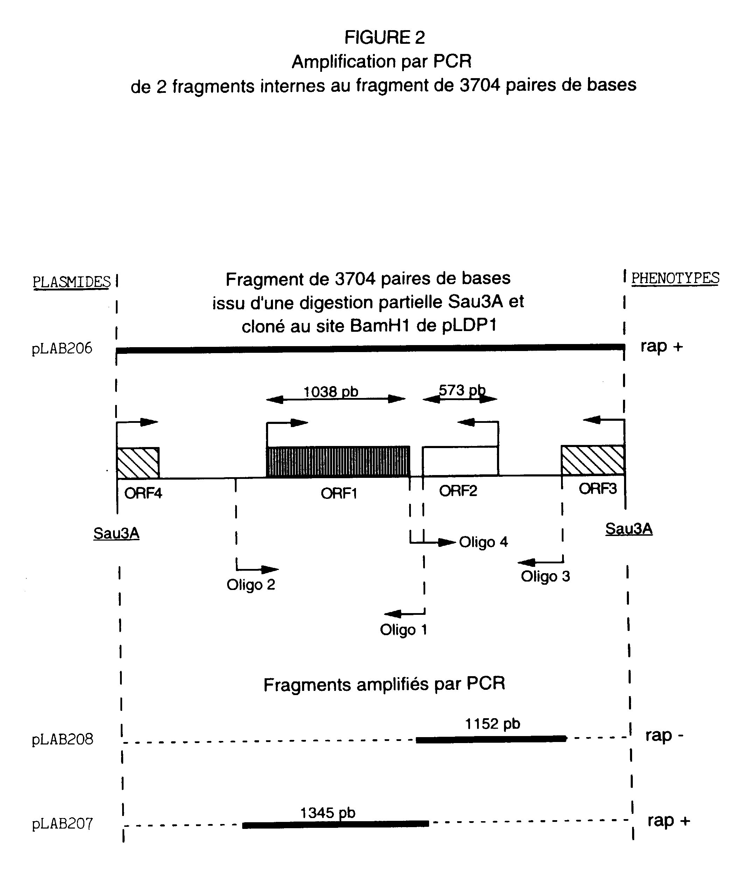

Leurs emplacements sur le fragment de 3704 pb sont indiqués sur la figure 2.

Les oligonucléotides N° 1 et 2 ont permis d'amplifier un fragment d'ADN de 1345 pb comportant l'ORF1 de 1038 pb sous la forme d'un fragment BamHI - SalI, grâce aux sites de restriction apportés par les oligonucléotides, permettant un clonage directionnel dans le plasmide navette pLDP1.

De la même manière, les oligonucléotides N° 3 et 4 ont permis d'amplifier un fragment d'ADN de 1152 pb comportant l'ORF2 de 573 pb sous forme d'un fragment BamHI-SalI comme ci-dessus.

Les fragments d'ADN ont été amplifiés par PCR à partir d'une préparation d'ADN total de la souche S94 (I-941) avec la Taq polymérase classique.

Les produits de PCR ont été purifiés par une extraction au phénol-chloroforme, précipités à l'éthanol, digérés par les enzymes de restriction BamHI et SalI et clones dans le vecteur navette pLDP1 ouvert aux sites BamHI et SalI.

Le clonage de ces fragments dans le vecteur pLDP1 a conduit aux plasmides recombinants pLAB207 et pLAB208. Ces plasmides ont été introduits dans la souche TG1 de E.coli et dans la souche S56 de L.lactis.

L'introduction des plasmides pLAB207 et pLAB208 dans la souche S56 de L.lactis a permis de déterminer s'ils confèrent la résistance aux phages.

Une synthèse des résultats concernant le clonage des différents fragments d'ADN amplifiés est présentée dans le tableau 1 suivant :

**TABLEAU 1**

| Paire d'oligonucléotides | Taille du fragment | Sites rajoutés | Cloné dans pLDP1 | Phénotypes* |
|---|---|---|---|---|
| 1-2 | 1345 pb | BamHI-SalI | pLAB207 | rap+ |
| 3-4 | 1152 pb | BamHI-SalI | pLAB208 | rap- |

| | | | | |
|---|---|---|---|---|
| * phénotypes : rap+ = résistance aux phages rap- = non résistance aux phages | | | | |

### Exemple 3 : Résistance aux phages conférée par le plasmide pLAB207

Le plasmide pLAB207 a été introduit dans la souche S56 L.lactis.

La résistance aux phages des clones obtenus a été testée en réalisant une titration (UFP/ml) avec les phages Ø 53 et Ø 59.

Les résultats sont donnés ci-après :

| Souche | phage Ø 53 (I) | | phage Ø 59 (III) | |
|---|---|---|---|---|
| | Titre (UFP/ml) | Taille des plages (mm) | Titre (UFP/ml) | Taille des plages (mm) |
| S56 | 10¹⁰ | 3 | 3.10⁹ | 2 |
| S56 (pLDP1) | 6.10⁹ | 3 | 2.10⁹ | 2 |
| S56 (pLAB207) | 10⁶ | 0,5 | 0 | - |
| UFP/ml = unité formant plages par ml | | | | |

Le plasmide pLAB207 contenant le fragment amplifié par PCR de 1345 pb correspondant à l'ORF1 confère une résistance aux phages.

## Revendications

1. Séquence d'acides nucléiques comprenant au moins un mécanisme de résistance aux phages, caractérisée en ce qu'elle est constituée par :
a) les séquences d'ADN présentant l'enchainement d'acides nucléiques [SEQ ID No. 1] ou [SEQ ID No. 3] ;
b) les séquences d'ADN hybridant avec les séquences ci-dessus ou un fragment de celles-ci ;
c) les séquences d'ARNm et d'ADNc correspondantes.

2. Séquence d'ADN comprenant au moins un mécanisme de résistance aux phages, caractérisée en ce qu'elle est choisie parmi les séquences [SEQ ID N° 1] et [SEQ ID N° 3] ou une séquence présentant un degré d'homologie élevée avec lesdites séquences [SEQ ID N° 1] et [SEQ ID N° 3].

3. Plasmide comprenant au moins un mécanisme de résistance aux phages caractérisé en ce qu'il comporte une séquence d'acides nucléiques selon la revendication 1.

4. Plasmide comprenant au moins un mécanisme de résistance aux phages caractérisé en ce qu'il comporte une séquence d'ADN selon la revendication 2.

5. Bactérie lactique résistante aux phages, caractérisée en ce qu'elle contient au moins un plasmide selon l'une des revendications 3 ou 4.

6. Utilisation des bactéries lactiques selon la revendication 5, pour conférer un mécanisme de résistance aux phages à des souches d'intérêt industriel.
